# EUROPEAN PATENT APPLICATION

(11) **EP 2 253 325 A1**
(43) Date of publication of application: **24.11.2010**
(21) Application number: 09714458.8
(22) Date of filing: 25.02.2009
(51) Int. Cl.: A61K 45/00, A61K 31/421, A61K 31/427, A61K 31/4439, A61K 31/517, A61P 27/02, A61P 31/04

(54) **AGENT FOR ENHANCING CORNEAL EPITHELIAL BARRIER FUNCTION**

(30) Priority: 25.02.2008 JP 2008043325
(71) Applicant: Santen Pharmaceutical Co., Ltd, Osaka-shi Osaka 533-8651 (JP)
(72) Inventor: HIRAI, Shin-ichiro, Ikoma-shi Nara 630-0101 (JP); YOSHIDA, Atsushi, Ikoma-shi Nara 630-0101 (JP)
(74) Representative: Lemcke, Brommer & Partner
(86) International application number: PCT/JP2009/053398
(87) International publication number: WO 2009/107652

(57) **Abstract**

The invention relates to a pharmacological effect of a compound functioning as PPARγ agonist on a corneal epithelial barrier function. Since the PPARγ agonist, such as rivoglitazone, DRF-2593, GW-544 and BMS-298585, exhibits an excellent enhancing effect on the corneal epithelial barrier function in an enhancement test of corneal epithelial barrier function, it is useful as a preventive or therapeutic agent for ocular infection, ocular discomfort and the like attributed to a decrease in the corneal epithelial barrier function. In addition, the PPARγ agonist can enhance the corneal epithelial barrier function in a diabetes patient, a patient with a decrease in the corneal epithelial barrier function due to aging, and a patient who underwent refractive surgery, such as PRK (photorefractive keratectomy) and LASIK (laser in situ keratomileusis), and cataract surgery.

## Description

### Technical field

The present invention relates to an agent for enhancing a corneal epithelial barrier function including a PPARγ agonist as active ingredient.

### Background art

A cornea is a transparent avascular tissue with a diameter of approximately 1 cm and a thickness of approximately 1 mm, is formed of corneal epithelium and corneal stroma, and has been known to have a significant effect on visual function.

As one of the important functions of the corneal epithelium, there can be mentioned a barrier formation between the ambient of the cornea and the corneal stroma. Such a corneal epithelial barrier function specifically is to regulate an invasion of substances present in lacrimal fluid, and pathogens, such as bacterium and fungus, from the corneal epithelium to the corneal stroma. It has been reported that the corneal epithelial barrier function is suppressed in a diabetes patient, and the corneal epithelial barrier function becomes reduced with aging (Non-Patent Documents 1, 2, and 3). It is believed that, when the barrier function is decreased, various substances present on the surface of eye disorderly invade the cornea and then stimulate a sensory nerve of the cornea. This stimulation becomes a factor of an ocular discomfort.

It has been also reported that, in the case of refractive surgery, such as PRK (photorefractive keratectomy) and LASIK (laser in situ keratomileusis), and cataract surgery, the corneal epithelial barrier function is reduced after the operation, and various pathogens invade the eye, which may cause infectious diseases (Non-Patent Documents 4 and 5). In addition, it is also known that, when the corneal epithelial barrier function collapse, an interaction between the corneal epithelium and the lacrimal fluid becomes disrupted, leading to lowering of functional visual acuity.

It has been known that PPARγ is distributed in energy storing organs, such as white adipose tissue, and has an effect on differentiation and proliferation of adipocyte. As a PPARγ agonist, there have been known compounds having a thiazolidinedione skeleton, such as 5-[4-(6-methoxy-1-methyl-1H-benzimidazol-2-yl methoxy)benzyl]thiazolidine-2,4-dione (rivoglitazone), 5-[4-[[3-methyl-4-oxo-3,4-dihydro-2-quinazolinyl]methoxy]phenylmethyl]thiazolidine-2, 4-dione (DRF-2593), 5-[[4-[2-(5-ethyl-2-pyridinyl)ethoxy]phenyl]methyl]-2,4-thiazolidinedione (pioglitazone), and 5-[p-[2-(methyl-2-pyridylamino)ethoxy]benzyl]-2,4-thiazolidinedione (rosiglitazone), and a compound having no thiazolidinedione skeleton, such as N-[1-methyl-3-oxo-3-phenyl-1(Z)-propenyl]-O-[2-(5-methyl-2-phenyloxazol-4-yl)ethyl]-L-tyrosine (GW-544), and N-[(4-methoxyphenoxy)carbonyl]-N-[[4-[2-(5-methyl-2-phenyl-4-oxazolyl)ethoxy]phenyl ]methyl]glycine (BMS-298585).

It has been reported that the PPARγ agonist improved insulin resistant diabetes, and was effective as therapeutic agent for disease attributed to insulin resistance, such as diabetes mellitus and hyperglycemia, as well as inflammatory disease, such as osteoarthritis and rheumatic arthritis (Patent Document 1). In addition, it has been also reported that the PPARγ agonist was effective as therapeutic agent for keratoconjunctive disorders, such as dry eye, corneal ulcer, keratitis, and conjunctivitis (Patent Documents 2, 3, and 4).

However, no report has been made with respect to the study of the above-described compound in terms of enhancing effect on the corneal epithelial barrier function. To examine a pharmacological effect of the compound functioning as PPARγ agonist on the corneal epithelial barrier function is very intriguing task.
Patent Document 1: Japanese Patent Publication No. 2976885
Patent Document 2: Japanese Patent Application Publication No. 2005-145961
Patent Document 3: Japanese Patent Application Publication No.2005-162735
Patent Document 4: Japanese Patent Application Publication No.2005-350451
Non-Patent Document 1: The Cornea. Scientific Foundations and Clinical Practice. Third Edition. (1994) 25-46
Non-Patent Document 2: Cornea 1993; 12(6): 493-499
Non-Patent Document 3: Cornea 2004; 23(1): 35-37
Non-Patent Document 4: Journal of Refractive Surgery 1999; 15 (2 suppl): S221-S224
Non-Patent Document 5: International Ophthalmology 1995-1996; 19(4): 225-233

### Disclosure of the invention

In order to examine the involvement of the PPARγ agonist in the corneal epithelial barrier function, the present inventors performed enhancement tests of corneal epithelial barrier function using compounds functioning as PPARγ agonist. As a result, the present inventors found that these compounds remarkably enhanced the corneal epithelial barrier function, and completed the present invention.

Accordingly, the present invention is directed to:
(1) an agent for enhancing the corneal epithelial barrier function that includes the PPARγ agonist as active ingredient,
(2) a preventive agent or therapeutic agent for ocular infection attributed to a decrease in the corneal epithelial barrier function that includes the PPARγ agonist as active ingredient,
(3) an agent for ameliorating an ocular discomfort attributed to a decrease in the corneal epithelial barrier function that includes the PPARγ agonist as active ingredient, and
(4) an agent for recovery of functional visual acuity that includes the PPARγ agonist as active ingredient.

In the present invention, there is no specific limitation with respect to the PPARγ agonist as long as it is a compound functioning as PPARγ agonist (hereinbelow, simply referred to as "present compound"), and it may be a compound having a thiazolidinedione skeleton or a compound having no thiazolidinedione skeleton. Examples of the compound having a thiazolidinedione skeleton includes,
5-[4-(6-methoxy-1-methyl-1H-benzimidazol-2-yl methoxy)benzyl]thiazolidine-2,4-dione (rivoglitazone),
5-[4-[[3-methyl-4-oxo-3,4-dihydro-2-quinazolinyl]methoxy]phenylmethyl]thiazolidine-2, 4-dione (DRF-2593),
5-[[4-[2-(5-ethyl-2-pyridinyl)ethoxy]phenyl]methyl]-2,4-thiazolidinedione (pioglitazone), and 5-[p-[2-(methyl-2-pyridylamino)ethoxy]benzyl]-2,4-thiazolidinedione (rosiglitazone) and examples of the compound having no thiazolidinedione skeleton includes
N-[1-methyl-3-oxo-3-phenyl-1(Z)-propenyl]-O-[2-(5-methyl-2-phenyloxazol-4-yl)ethyl]-L-tyrosine (GW-544),
N-[(4-methoxyphenoxy)carbonyl]-N-[[4-[2-(5-methyl-2-phenyl-4-oxazolyl)ethoxy]phenyl ]methyl]glycine (BMS-298585),
E-4-[4-(5-methyl-2-phenyl-4-oxazolylmethoxy)benzyloximino]-4-phenyl butyric acid (TAK-559),
Z-2-[4-(5-methyl-2-phenyl-4-oxazolylmethoxy)benzyloximino]-2-(4-phenoxyphenyl) acetic acid (TAK-664),
2-[2-propyl-3-[3-[2-ethyl-4-(4-fluorophenyl)-5-hydroxyphenoxy]propoxy]phenoxy] benzoic acid (LY-2931111),
2(S)-methoxy-3-[4-[3-(4-phenoxyphenoxy)propoxy]phenyl]propionic acid (LY-519818), (-)-2-(4-chlorophenyl)-2-[3-(trifluoromethyl)phenoxy]acetic acid 2-(acetylamino)ethyl ester (MBX-102), and (-)-2-(4-chlorophenyl)-2-[3-(trifluoromethyl)phenoxy]acetic acid.

With respect to the salt of the compound described above, there is no limitation as long as it is pharmaceutically compatible, and examples include: a salt with inorganic acid, such as hydrochloric acid, nitric acid and sulfuric acid; a salt with organic acid, such as acetic acid, fumaric acid, maleic acid, succinic acid and tartaric acid; and a salt with alkali metal or alkali earth metal, such as sodium, potassium and calcium. A preferable salt is a salt of hydrochloric acid. In addition, a quaternary ammonium salt of the present compound is included in the salt in the present invention. Further, when a geometric isomer or an optical isomer of the present compound is present, the isomer thereof is also included in the present invention. It should be noted that the present compound may be in a form of hydrate or solvate.

In the present invention, the expression "corneal epithelial barrier function" refers to a function of regulating an invasion of substances present in lacrimal fluid, and an invasion of pathogens, such as bacterium and fungus, from the corneal epithelium to the corneal stroma. The agent for enhancing the corneal epithelial barrier function of the present invention can enhance the corneal epithelial barrier function in a diabetes patient, a patient with a decrease in the corneal epithelial barrier function due to aging, and a patient who underwent refractive surgery, such as PRK (photorefractive keratectomy) and LASIK (laser in situ keratomileusis), and cataract surgery.

By enhancing the corneal epithelial barrier function, for example, it becomes possible to prevent or treat ocular infection attributed to a decrease in the corneal epithelial barrier function, to ameliorate the ocular discomfort attributed to a decrease in the corneal epithelial barrier function, and to repair the disrupted interaction between the corneal epithelium and the lacrimal fluid to prevent lowering of functional visual acuity.

The agent for enhancing the corneal epithelial barrier function of the present invention may be administered orally or parenterally. Examples of types of administration include eye-drop, eye ointment, injectable, tablet, capsule, granule, and powder, and especially the eye-drop is preferred. These can be pharmaceutically formulated using common techniques. For example, in the case of eye-drop, a pharmaceutically formulation can be prepared using, if desired, isotonic agent, such as sodium chloride and concentrated glycerin; buffer agent, such as sodium phosphate and sodium acetate; surfactant, such as polyoxyethylene sorbitan monooleate, polyoxyl 40 stearate, and polyoxyethylene hardened castor oil; stabilizing agent, such as sodium citrate and sodium edetate; and preservative agent, such as benzalkonium chloride and paraben. There is no limitation with respect to pH as long as it is compatible with ophthalmologic preparation, but a range of 4 - 8 is preferable.

In the case of the eye ointment, it can be prepared using common ointment base, such as white petrolatum and liquid paraffin. In the case of the oral agent, such as tablet, capsule, granule, and powder, if desired, there may be added: expander, such as lactose, crystalline cellulose, starch and vegetable oil; lubricant, such as magnesium stearate and talc; binder, such as hydroxylpropyl cellulose and polyvinylpyrrolidone; disintegrant, such as carboxy methylcellulose calcium and hydroxypropyl methylcellulose of low substitution; coating agent, such as hydroxypropyl methylcellulose, macrogol, and silicon resin; and film-forming agent, such as gelatin film.

The dosage can be appropriately selected depending on symptoms, age, dosage form and the like, but it is preferred that, in the case of the eye-drop, a drop with 0.0001 - 1 %(w/v), preferably 0.001 - 1% (w/v) is placed in the eye once or several times per day. In the case of the oral agent, in general, 0.1 - 5,000 mg, preferably 1 - 1,000 mg thereof is administered once or several times per day.

As will be described later, the enhancement test of corneal epithelial barrier function elucidated that the compound of the present invention functioning as PPARγ agonist exhibited an excellent enhancing effect on the barrier function. Accordingly, it becomes possible to prevent or treat ocular infection attributed to a decrease in the corneal epithelial barrier function, to ameliorate the ocular discomfort attributed to a decrease in the corneal epithelial barrier function, and to repair the disrupted interaction between the corneal epithelium and the lacrimal fluid to prevent lowering of functional visual acuity. In addition, the compound of the present invention can enhance the corneal epithelial barrier function in a diabetes patient, a patient with a decrease in the corneal epithelial barrier function due to aging, and a patient with refractive surgery, such as PRK (photorefractive keratectomy) and LASIK (laser in situ keratomileusis), and cataract surgery.

### Best mode for carrying out the invention

Results of the pharmacological tests and embodiments of pharmaceutical preparations will be described below. These are illustrated for the purpose of helping better understanding of the present invention, and it should not be construed that these limit the scope of the present invention.

### [Pharmacological test 1] Enhancing effect of PPARγ agonist on barrier function

The PPARγ agonist was evaluated with respect to the enhancing effect on the barrier function of a corneal epithelial cell. For the corneal epithelial cell, an SV40-immortalized human corneal epithelial cell line was used, utilizing a membrane electrical resistance as an index of the barrier function. When the barrier function of the corneal epithelial cell is enhanced, the membrane electrical resistance increases, while the barrier function is reduced, the membrane electrical resistance decreases.

### (Experimental methodology)

From RIKEN BioResource Center, SV40-immortalized human corneal epithelial cells (HCE-T) were obtained. On a culture insert of 24-well Transwell Clear (Coming incorporated) were seeded 6.4×10⁴ cells of HCE-T, and incubated at 37°C in 5% CO₂. As the culture medium, a DMEM/Ham's F12 (Nacalai Tesque, Inc.) containing 15% fetal bovine serum (ICN) and 40 µg/mL of gentamicin (Gibco) was used. The culture medium was removed after 9 hours, 2 days, 3 days and 4 days, and replaced with a culture medium containing one of
5-[4-(6-methoxy-1-methyl-1H-benzimidazol-2-ylmethoxy)benzyl]thiazolidine-2,4-dione (rivoglitazone),
5-[4-[[3-methyl-4-oxo-3,4-dihydro-2-quinazolinyl]methoxy]phenylmethyl]thiazolidine-2, 4-dione (DRF-2593),
5-[[4-[2-(5-ethyl-2-pyridinyl)ethoxy]phenyl]methyl]-2,4-thiazolidinedione (pioglitazone), and 5-[p-[2-(methyl-2-pyridylamino)ethoxy]benzyl]-2,4-thiazolidinedione (rosiglitazone), as PPARγ agonist with a thiazolidinedione skeleton, each in an amount of 10 µM, or a culture medium containing one of
N-[1-methyl-3-oxo-3-phenyl-1(Z)-propenyl]-O-[2-(5-methyl-2-phenyloxazol-4-yl)ethyl]-L-tyrosine (GW-544), and
N-[(4-methoxyphenoxy)carbonyl]-N-[[4-[2-(5-methyl-2-phenyl-4-oxazolyl)ethoxy]phenyl ]methyl]glycine (BMS-298585), as PPARγ agonist with a non-thiazolidinedione skeleton, each in an amount of 10 µM, to thereby obtain samples. The concentration mentioned above was attained by dissolving each sample in DMSO and diluting 1,000-fold with the culture medium. In addition, a culture medium containing DMSO alone was used as a base culture medium.

Five days after the seeding, an electrical resistance of an epithelial cell layer was measured using a membrane electrical resistance measurement system (World Precision Instruments). A well containing the base medium alone with no corneal epithelial cell seeded was measured as a blank, and the value was subtracted from the electrical resistance of the epithelial cell layer. The resultant value was multiplied by an area of the cell culture (0.33 cm²), to thereby obtain an electrical resistance (Ω•cm²) per unit area.

### (Result)

The membrane electrical resistance (average of four experiments) of each sample is shown in Table 1.

**[Table 1]**

| Sample [10 µM] | Membrane electrical resistance (Ω•cm²) |
|---|---|
| Rivoglitazone | 615.9 |
| DRF-2593 | 485.8 |
| Pioglitazone | 350.3 |
| Rosiglitazone | 343.2 |
| GW-544 | 435.3 |
| BMS-298585 | 375.1 |
| Base culture | 296.0 |

### (Considerations)

As is apparent from Table 1, all of the PPARγ agonists used in the present pharmacological test showed higher electrical resistance than that of the base culture medium. In other words, it was shown that the PPARγ agonist, regardless of the presence of the thiazolidinedione skeleton, enhanced the barrier function of the corneal epithelial cell. Especially, rivoglitazone, DRF-2593, BMS-298585, and GW-544 showed an excellent barrier function enhancing effect, and still especially rivoglitazone exhibited an extremely strong barrier function enhancing effect.

### [Pharmacological test 2] Influence of PPARγ antagonist on barrier function enhancing effect of PPARγ agonist

An influence of the PPARγ antagonist was evaluated with respect to the barrier function enhancing effect of the PPARγ agonist, and it was examined whether or not the enhancing effect on the barrier function is based on the activity of the PPARγ agonist.

### (Experimental methodology)

From RIKEN BioResource Center, SV40-immortalized human corneal epithelial cells (HCE-T) were obtained. On a culture insert of 24-well Transwell Clear (Coming Incorporated) were seeded 6.4×10⁴ cells of HCE-T, and incubated at 37°C in 5%CO₂. As the culture medium, a DMEM/Ham's F12 (Nacalai Tesque, Inc.) containing 15% fetal bovine serum (ICN) and 40 µg/mL of gentamicin (Gibco) was used. The culture medium was removed after 9 hours, 2 days, 3 days and 4 days, and replaced with a culture medium containing the PPARγ agonist and the PPARγ antagonist to thereby obtain samples. As the PPARγ agonist, rivoglitazone [0.1 µM] was used, and as the PPARγ antagonist, 2-chloro-5-nitrobenzanilide (GW-9662) [0.03 µM, 0.1 µM] was used. The concentrations mentioned above were attained by dissolving each sample in DMSO and diluting 1,000-fold with the culture medium. In addition, a culture containing DMSO alone was used as a base culture medium.

Five days after the seeding, an electrical resistance of an epithelial cell layer was measured using a membrane electrical resistance measurement system (World Precision Instruments). A well containing the base medium alone with no corneal epithelial cell seeded was measured as a blank, and the value was subtracted from the electrical resistance of the epithelial cell layer. The resultant value was multiplied by an area of the cell culture (0.33 cm²), to thereby obtaining an electrical resistance (Ω•cm²) per unit area.

### (Result)

The membrane electrical resistance (average of four experiments) of each sample is shown in Table 2.

**[Table 2]**

| Sample | Membrane electrical resistance (Ω•cm²) |
|---|---|
| Rivoglitazone (0.1 µM) | 538.8 |
| Rivoglitazone (0.1 µM) and GW-9662 (0.03 µM) | 473.5 |
| Rivoglitazone (0.1 µM) and GW-9662 (0.1 µM) | 429.3 |
| Base culture | 434.1 |

### (Considerations)

As is apparent from Table 2, the barrier function enhanced by rivoglitazone (PPARγ agonist) was concentration-dependently reduced by GW-9662 (PPARγ antagonist), and completely antagonized at 0.1 µM. In other words, it was shown that the enhancing effect of rivoglitazone on the corneal epithelial cell barrier function is PPARγ-dependent. The results above elucidate that, when the compound has the PPARγ agonist activity, the compound can enhance the barrier function of the corneal epithelial cell.

### [Examples of pharmaceutical formulation]

Representative pharmaceutical formulations using the PPARγ agonist will be described below.

### Formulation Example 1

In 100 ml

| | |
|---|---|
| Rivoglitazone | : 10 mg |
| Sodium chloride | : 900 mg |
| Sterile purified water | : appropriate amount |

By altering the amount of rivoglitazone, eye-drop with various concentrations, such as 0.001%(w/v), 0.01%(w/v), 0.03%(w/v), 0.1 %(w/v), 0.3%(w/v), 1.0%(w/v), and 3.0%(w/v), can be prepared.

### Formulation Example 2

In 100 ml

| | |
|---|---|
| DRF-2593 | : 100 mg |
| Sodium chloride | : 800 mg |
| Disodium hydrogenphosphate | : 100 mg |
| Sodium dihydrogenphosphate | : appropriate amount |
| Sterile purified water | : appropriate amount |

By altering the amount of DRF-2593, eye-drop with various concentrations, such as 0.1%(w/v), 0.3%(w/v), 0.5%(w/v), 1.5%(w/v), and 3%(w/v), can be prepared.

### Formulation Example 3

In 100g

| | |
|---|---|
| Rivoglitazone | : 0.3g |
| Liquid paraffin | : 10.0g |
| White petrolatum | : appropriate amount |

By altering the amount of rivoglitazone, eye ointment with various concentrations, such as 1%(w/w) and 3%(w/w), can be prepared.

### Formulation Example 4

In 100g

| | |
|---|---|
| GW-544 | : 0.3g |
| Liquid paraffin | : 10.0g |
| White petrolatum | : appropriate amount |

By altering the amount of GW-544, eye ointment with various concentrations, such as 1%(w/w) and 3%(w/w), can be prepared.

### Industrial applicability

The compound functioning as the PPARγ agonist remarkably enhances the corneal epithelial barrier function. By enhancing the corneal epithelial barrier function, for example, it becomes possible to prevent or treat ocular infection attributed to a decrease in the corneal epithelial barrier function, to ameliorate the ocular discomfort attributed to a decrease in the corneal epithelial barrier function, and to repair the disrupted interaction between the corneal epithelium and the lacrimal fluid to prevent lowering of functional visual acuity.

## Claims

1. An agent for enhancing a corneal epithelial barrier function comprising a PPARγ agonist as active ingredient.

2. The agent for enhancing a corneal epithelial barrier function according to claim 1, wherein the PPARγ agonist is at least one of
5-[4-(6-methoxy-1-methyl-1H-benzimidazol-2-yl methoxy)benzyl]thiazolidine-2,4-dione (rivoglitazone),
5-[4-[[3-methyl-4-oxo-3,4-dihydro-2-quinazolinyl]methoxy]phenylmethyl]thiazolidine-2, 4-dione (DRF-2593),
5-[[4-[2-(5-ethyl-2-pyridinyl)ethoxy]phenyl]methyl]-2,4-thiazolidinedione (pioglitazone),
5-[p-[2-(methyl-2-pyridylamino)ethoxy]benzyl]-2,4-thiazolidinedione (rosiglitazone),
N-[1-methyl-3-oxo-3-phenyl-1(Z)-propenyl]-O-[2-(5-methyl-2-phenyloxazol-4-yl)ethyl]-L-tyrosine (GW-544),
N-[(4-methoxyphenoxy)carbonyl]-N-[[4-[2-(5-methyl-2-phenyl-4-oxazolyl)ethoxy]phenyl ]methyl]glycine (BMS-298585), and a salt thereof.

3. A preventive or therapeutic agent for ocular infection attributed to a decrease in a corneal epithelial barrier function comprising a PPARγ agonist as active ingredient.

4. The preventive or therapeutic agent for ocular infection attributed to a decrease in a corneal epithelial barrier function according to claim 3, wherein the PPARγ agonist is at least one of 5-[4-(6-methoxy-1-methyl-1H-benzimidazol-2-yl methoxy)benzyl]thiazolidirie-2,4-dione (rivoglitazone),
5-[4-[[3-methyl-4-oxo-3,4-dihydro-2-quinazolinyl]methoxy]phenylmethyl]thiazolidine-2, 4-dione (DRF-2593),
5-[[4-[2-(5-ethyl-2-pyridinyl)ethoxy]phenyl]methyl]-2,4-thiazolidinedione (pioglitazone),
5-[p-[2-(methyl-2-pyridylamino)ethoxy]benzyl]-2,4-thiazolidinedione (rosiglitazone),
N-[1-methyl-3-oxo-3-phenyl-1(Z)-propenyl]-O-[2-(5-methyl-2-phenyloxazol-4-yl)ethyl]-L-tyrosine (GW-544),
N-[(4-methoxyphenoxy)carbonyl]-N-[[4-[2-(5-methyl-2-phenyl-4-oxazolyl)ethoxy]phenyl ]methyl]glycine (BMS-298585), and a salt thereof.

5. An agent for ameliorating ocular discomfort attributed to a decrease in a corneal epithelial barrier function comprising a PPARγ agonist as active ingredient.

6. The ameliorating agent for ocular discomfort attributed to a decrease in a corneal epithelial barner function according to claim 5, wherein the PPARγ agonist is at least one of 5-[4-(6-methoxy-1-methyl-1H-benzimidazol-2-yl methoxy)benzyl]thiazolidine-2,4-dione (rivoglitazone),
5-[4-[[3-methyl-4-oxo-3,4-dihydro-2-quinazolinyl]methoxy]phenylmethyl]thiazolidine-2,
4-dione (DRF-2593),
5-[[4-[2-(5-ethyl-2-pyridinyl)ethoxy]phenyl]methyl]-2,4-thiazolidinedione (pioglitazone),
5-[p-[2-(methyl-2-pyridylamino)ethoxy]benzyl]-2,4-thiazolidinedione (rosiglitazone),
N-[1-methyl-3-oxo-3-phenyl-1(Z)-propenyl]-O-[2-(5-methyl-2-phenyloxazol-4-yl)ethyl]-L-tyrosine (GW-544),
N-[(4-methoxyphenoxy)carbonyl]-N-[[4-[2-(5-methyl-2-phenyl-4-oxazolyl)ethoxy]phenyl ]methyl]glycine (BMS-298585), and a salt thereof.

7. An agent for recovery of functional visual acuity comprising a PPARγ agonist as active ingredient.

8. The agent for recovery of functional visual acuity according to claim 7, wherein the PPARγ agonist is at least one of 5-[4-(6-methoxy-1-methyl-1H-benzimidazol-2-yl methoxy)benzyl]thiazolidine-2,4-dione (rivoglitazone),
5-[4-[[3-methyl-4-oxo-3,4-dihydro-2-quinazolinyl]methoxy]phenylmethyl]thiazolidine-2,
4-dione (DRF-2593),
5-[[4-[2-(5-ethyl-2-pyridinyl)ethoxy]phenyl]methyl]-2,4-thiazolidinedione (pioglitazone),
5-[p-[2-(methyl-2-pyridylamino)ethoxy]benzyl]-2,4-thiazolidinedione (rosiglitazone),
N-[1-methyl-3-oxo-3-phenyl-1(Z)-propenyl]-O-[2-(5-methyl-2-phenyloxazol-4-yl)ethyl]-L-tyrosine (GW-544),
N-[(4-methoxyphenoxy)carbonyl]-N-[[4-[2-(5-methyl-2-phenyl-4-oxazolyl)ethoxy]phenyl ]methyl]glycine (BMS-298585), and a salt thereof.

9. A therapeutic method for a disorder attributed to a decrease in a corneal epithelial barrier function comprising a step of administering a pharmacologically effective amount of a PPARγ agonist to a patient.

10. The therapeutic method for a disorder attributed to a decrease in a corneal epithelial barrier function according to claim 9, wherein the PPARγ agonist is at least one of 5-[4-(6-methoxy-1-methyl-1H-benzimidazol-2-yl methoxy)benzyl]thiazolidine-2,4-dione (rivoglitazone),
5-[4-[[3-methyl-4-oxo-3,4-dihydro-2-quinazolinyl]methoxy]phenylinethyl]thiazolidine-2,
4-dione (DRF-2593),
5-[[4-[2-(5-ethyl-2-pyridinyl)ethoxy]phenyl]methyl]-2,4-thiazolidinedione (pioglitazone),
5-[p-[2-(methyl-2-pyridylamino)ethoxy]benzyl]-2,4-thiazolidinedione (rosiglitazone),
N-[1-methyl-3-oxo-3-phenyl-1(Z)-propenyl]-O-[2-(5-methyl-2-phenyloxazol-4-yl)ethyl]-L-tyrosine (GW-544),
N-[(4-methoxyphenoxy)carbonyl]-N-[[4-[2-(5-methyl-2-phenyl-4-oxazolyl)ethoxy]phenyl ]methyl]glycine (BMS-298585), and a salt thereof.

11. A preventive or therapeutic method for ocular infection attributed to a decrease in a corneal epithelial barrier function comprising a step of administering a pharmacologically effective amount of a PPARγ agonist to a patient.

12. The preventive or therapeutic method for ocular infection attributed to a decrease in a corneal epithelial barrier function according to claim 11, wherein the PPARγ agonist is at least one of 5-[4-(6-methoxy-1-methyl-1H-benzimidazol-2-yl methoxy)benzyl]thiazolidine-2,4-dione (rivoglitazone),
5-[4-[[3-methyl-4-oxo-3,4-dihydro-2-quinazolinyl]methoxy]phenylmethyl]thiazolidine-2,
4-dione (DRF-2593),
5-[[4-[2-(5-ethyl-2-pyridinyl)ethoxy]phenyl]methyl]-2,4-thiazolidinedione (pioglitazone),
5-[p-[2-(methyl-2-pyridylamino)ethoxy]benzyl]-2,4-thiazolidinedione (rosiglitazone),
N-[1-methyl-3-oxo-3-phenyl-1(Z)-propenyl]-O-[2-(5-methyl-2-phenyloxazol-4-yl)ethyl]-L-tyrosine (GW-544),
N-[(4-methoxyphenoxy)carbonyl]-N-[[4-[2-(5-methyl-2-phenyl-4-oxazolyl)ethoxy]phenyl ]methyl]glycine (BMS-298585), and a salt thereof.

13. A therapeutic method for ocular discomfort attributed to a decrease in a corneal epithelial barrier function comprising a step of administering a pharmacologically effective amount of a PPARγ agonist to a patient.

14. The therapeutic method for ocular discomfort attributed to a decrease in a corneal epithelial barrier function according to claim 13, wherein the PPARγ agonist is at least one of 5-[4-(6-methoxy-1-methyl-1H-benzimidazol-2-yl methoxy)benzyl]thiazolidine-2,4-dione (rivoglitazone),
5-[4-[[3-methyl-4-oxo-3,4-dihydro-2-quinazolinyl]methoxy]phenylmethyl]thiazolidine-2, 4-dione (DRF-2593),
5-[[4-[2-(5-ethyl-2-pyridinyl)ethoxy]phenyl]methyl]-2,4-thiazolidinedione (pioglitazone),
5-[p-[2-(methyl-2-pyridylamino)ethoxy]benzyl]-2,4-thiazolidinedione (rosiglitazone),
N-[1-methyl-3-oxo-3-phenyl-1(Z)-propenyl]-O-[2-(5-methyl-2-phenyloxazol-4-yl)ethyl]-L-tyrosine (GW-544),
N-[(4-methoxyphenoxy)carbonyl]-N-[[4-[2-(5-methyl-2-phenyl-4-oxazolyl)ethoxy]phenyl ]methyl]glycine (BMS-298585), and a salt thereof.

15. A therapeutic method for recovering functional visual acuity comprising a step of administering a pharmacologically effective amount of a PPARγ agonist to a patient.

16. The therapeutic method for recovering functional visual acuity according to claim 15, wherein the PPARγ agonist is at least one of
5-[4-(6-methoxy-1-methyl-1H-benzimidazol-2-yl methoxy)benzyl]thiazolidine-2,4-dione (rivoglitazone), 5-[4-[[3-methyl-4-oxo-3,4-dihydro-2-quinazolinyl]methoxy]phenylmethyl]thiazolidine-2,
4-dione (DRF-2593),
5-[[4-[2-(5-ethyl-2-pyridinyl)ethoxy]phenyl]methyl]-2,4-thiazolidinedione (pioglitazone),
5-[p-[2-(methyl-2-pyridylamino)ethoxy]benzyl]-2,4-thiazolidinedione (rosiglitazone),
N-[1-methyl-3-oxo-3-phenyl-1(Z)-propenyl]-O-[2-(5-methyl-2-phenyloxazol-4-yl)ethyl]-L-tyrosine (GW-544),
N-[(4-methoxyphenoxy)carbonyl]-N-[[4-[2-(5-methyl-2-phenyl-4-oxazolyl)ethoxy]phenyl ]methyl]glycine (BMS-298585), and a salt thereof.

17. A use of a PPARγ agonist for producing an agent for enhancing a corneal epithelial barrier function.

18. The use of a PPARγ agonist for producing an agent for enhancing a corneal epithelial barrier function according to claim 17, wherein the PPARγ agonist is at least one of 5-[4-(6-methoxy-1-methyl-1H-benzimidazol-2-yl methoxy)benzyl]thiazolidine-2,4-dione (rivoglitazone),
5-[4-[[3-methyl-4-oxo-3,4-dihydro-2-quinazolinyl]methoxy]phenylinethyl]thiazolidine-2,
4-dione (DRF-2593),
5-[[4-[2-(5-ethyl-2-pyridinyl)ethoxy]phenyl]methyl]-2,4-thiazolidinedione (pioglitazone),
5-[p-[2-(methyl-2-pyridylamino)ethoxy]benzyl]-2,4-thiazolidinedione (rosiglitazone),
N-[1-methyl-3-oxo-3-phenyl-1(Z)-propenyl]-O-[2-(5-methyl-2-phenyloxazol-4-yl)ethyl]-L-tyrosine (GW-544),
N-[(4-methoxyphenoxy)carbonyl]-N-[[4-[2-(5-methyl-2-phenyl-4-oxazolyl)ethoxy]phenyl ]methyl]glycine (BMS-298585), and a salt thereof.

19. A use of a PPARγ agonist for producing a preventive or therapeutic agent for ocular infection attributed to a decrease in a corneal epithelial barrier function.

20. The use of a PPARγ agonist for producing a preventive or therapeutic agent for ocular infection attributed to a decrease in a corneal epithelial barrier function according to claim 19, wherein the PPARγ agonist is at least one of
5-[4-(6-methoxy-1-methyl-1H-benzimidazol-2-yl methoxy)benzyl]thiazolidine-2,4-dione (rivoglitazone),
5-[4-[[3-methyl-4-oxo-3,4-dihydro-2-quinazolinyl]methoxy]phenylinethyl]thiazolidine-2, 4-dione (DRF-2593),
5-[[4-[2-(5-ethyl-2-pyridinyl)ethoxy]phenyl]methyl]-2,4-thiazolidinedione (pioglitazone),
5-[p-[2-(methyl-2-pyridylamino)ethoxy]benzyl]-2,4-thiazolidinedione (rosiglitazone),
N-[1-methyl-3-oxo-3-phenyl-1(Z)-propenyl]-O-[2-(5-methyl-2-phenyloxazol-4-yl)ethyl]-L-tyrosine (GW-544),
N-[(4-methoxyphenoxy)carbonyl]-N-[[4-[2-(5-methyl-2-phenyl-4-oxazolyl)ethoxy]phenyl ]methyl]glycine (BMS-298585), and a salt thereof.

21. A use of a PPARγ agonist for producing an ameliorating agent for ocular discomfort attributed to a decrease in a corneal epithelial barrier function.

22. The use of a PPARγ agonist for producing an ameliorating agent for ocular discomfort attributed to a decrease in a corneal epithelial barrier function according to claim 21, wherein the PPARγ agonist is at least one of
5-[4-(6-methoxy-1-methyl-1H-benzimidazol-2-yl methoxy)benzyl]thiazolidine-2,4-dione (rivoglitazone),
5-[4-[[3-methyl-4-oxo-3,4-dihydro-2-quinazolinyl]methoxy]phenylmethyl]thiazolidine-2, 4-dione (DRF-2593),
5-[[4-[2-(5-ethyl-2-pyridinyl)ethoxy]phenyl]methyl]-2,4-thiazolidinedione (pioglitazone),
5-[p-[2-(methyl-2-pyridylamino)ethoxy]benzyl]-2,4-thiazolidinedione (rosiglitazone),
N-[1-methyl-3-oxo-3-phenyl-1(Z)-propenyl]-O-[2-(5-methyl-2-phenyloxazol-4-yl)ethyl]-L-tyrosine (GW-544),
N-[(4-methoxyphenoxy)carbonyl]-N-[[4-[2-(5-methyl-2-phenyl-4-oxazolyl)ethoxy]phenyl ]methyl]glycine (BMS-298585), and a salt thereof.

23. A use of a PPARγ agonist for producing an agent for recovery of functional visual acuity.

24. The use of a PPARγ agonist for producing the agent for recovery of functional visual acuity according to claim 23, wherein the PPARγ agonist is at least one of
5-[4-(6-methoxy-1-methyl-1H-benzimidazol-2-yl methoxy)benzyl]thiazolidine-2,4-dione (rivoglitazone),
5-[4-[[3-methyl-4-oxo-3,4-dihydro-2-quinazolinyl]methoxy]phenylmethyl]thiazolidine-2, 4-dione (DRF-2593),
5-[[4-[2-(5-ethyl-2-pyridinyl)ethoxy]phenyl]methyl]-2,4-thiazolidinedione (pioglitazone),
5-[p-[2-(methyl-2-pyridylamino)ethoxy]benzyl]-2,4-thiazolidinedione (rosiglitazone),
N-[1-methyl-3-oxo-3-phenyl-1(Z)-propenyl]-O-[2-(5-methyl-2-phenyloxazol-4-yl)ethyl]-L-tyrosine (GW-544),
N-[(4-methoxyphenoxy)carbonyl]-N-[[4-[2-(5-methyl-2-phenyl-4-oxazolyl)ethoxy]phenyl ]methyl]glycine (BMS-298585), and a salt thereof.
